# EUROPEAN PATENT APPLICATION

(11) **EP 2 746 293 A1**
(43) Date of publication of application: **25.06.2014**
(21) Application number: 13198694.5
(22) Date of filing: 20.12.2013
(51) Int. Cl.: C07K 16/24, A61P 9/10

(54) **Anti-IL21 antibodies for use in the treatment of cardiovascular diseases**

(30) Priority: 21.12.2012 EP 12198937; 16.01.2013 US 201361753125 P
(71) Applicant: Novo Nordisk A/S, 2880 Bagsværd (DK)
(72) Inventor: Junker, Nanna, 2850 Naerum (DK); Cucak, Helena, 218 53 Malmö (DK); Rosendahl, Alexander, 218 53 Klagshamn (SE)

(57) **Abstract**

The present invention relates to treatment of cardiovascular diseases and arthero-sclerosis. In particular, the present invention relates to use of therapeutic antibodies.

## Description

### TECHNICAL FIELD

The present invention relates to treatment of cardiovascular disease and atherosclerosis/arthero-sclerosis in humans. In particular, the present invention relates to treatment of such diseases using therapeutic antibodies.

### BACKGROUND

Cardiovascular diseases (CVD) remain the biggest cause of deaths worldwide. Although cardiovascular disease usually affects older adults, the antecedants of cardiovascular disease, notably athero-sclerosis, begin in early life and thus making primary prevention efforts necessary. There is therefore increased emphasis on preventing atherosclerosis by modifying risk factors, such as healthy eating, exercise, and avoidance of smoking. Athero-sclerosis is a condition in which an artery wall thickens as a result of the accumulation of fatty materials such as cholesterol. It is a syndrome affecting arterial blood vessels, a chronic inflammatory response in the walls of arteries, caused largely by the accumulation of macrophage white blood cells and promoted by low-density lipoproteins (LDL, plasma proteins that carry cholesterol and triglycerides) without adequate removal of fats and cholesterol from the macrophages by functional high-density lipoproteins (HDL). It is commonly referred to as a hardening or furring of the arteries. It is caused by the formation of multiple "plaques" within the arteries. Inflammation is considered to be a cause of atherosclerosis rather than intake of fat and cholesterol

Diabetes mellitus is a major risk factor for athero-sclerosis and CVD. High blood sugar has two effects on cells lining blood vessels as part of athero-sclerosis. First, it increases the production of free radicals, causing premature cell death (apoptosis). This process also reduces the availability of nitric oxide (NO), which would otherwise enable blood vessels to relax and blood flow to increase.

Inflammation in blood vessels is one of the main drivers of athero-sclerosis, and diabetes thus makes it worse. Hyperglycemia-induced endothelial dysfunction accelerates the process of athero-thrombotic complications. Lipid accumulation in the area of the atherosclerotic lesion is primarily in the form of intracellular and extracellular esterified cholesterol. In uncontrolled or poorly controlled diabetes, elevated plasma low density lipoprotein levels and decreased plasma high density lipoprotein levels favor lipid deposition in large vessels.

Cardiovascular diseases, such as arthero-sclerosis, are currently treated with aspirin, ACE inhibitors, statins, and other drugs that may lower the risk of cardiovascular mortality by reducing progression of arthero-sclerosis. There is thus a great need in the art for alternative and/or improved treatment options of this serious chronic condition.

### SUMMARY

The present invention relates to use of IL-21/IL-21Rα antagonists for treatment of cardiovascular diseases. Such drugs have the potential to provide alternative and preferably improved treatment options for cardiovascular disease patients.

### DESCRIPTION

### Definitions:

"Cardiovascular diseases" (CVD) refers to chronic heart and blood vessel diseases and includes numerous problems, many of which are related to arthero-sclerosis. "Atherosclerosis" is a chronic disease that normally remains asymptomatic for decades. Atherosclerotic lesions/plaques are separated into two broad categories: Stable and unstable (also called vulnerable). "Stable plaques", tend to be asymptomatic, and are rich in extracellular matrix and smooth muscle cells, while, "unstable plaques" are rich in macrophages and foam cells and the extracellular matrix separating the lesion from the arterial lumen (also known as the fibrous cap) is usually weak and prone to rupture. Rupture of the unstable "cap" leads to exposure of thrombogenic material, such as e.g. collagen, to the circulation and eventually thrombus formation in the lumen. Upon formation, intraluminal thrombi can occlude arteries outright (i.e. coronary occlusion), but more often they detach, move into the circulation and eventually occlude smaller downstream branches causing thrombo-embolism. Apart from thrombo-embolism, chronically expanding athero-sclerotic lesions can cause complete closure of the lumen. Chronically expanding lesions are often asymptomatic until lumen stenosis is so severe that blood supply to downstream tissue(s) is insufficient - resulting in ischemia.

These complications of advanced athero-sclerosis are chronic, slowly progressing and cumulative. Most commonly, soft plaques suddenly ruptures, causing the formation of a thrombus that will rapidly slow or stop blood flow, leading to death of the tissues fed by the artery in approximately 5 minutes. This catastrophic event is called an infarction. One of the most common recognized scenarios is called coronary thrombosis of a coronary artery, causing myocardial infarction (a heart attack). The same process in an artery to the brain is commonly called stroke. Another common scenario in very advanced disease is claudication from insufficient blood supply to the legs, typically caused by a combination of both stenosis and aneurysmal segments narrowed with clots.

Athero-sclerosis affects the entire artery tree, but mostly larger, high-pressure vessels such as the coronary, renal, femoral, cerebral, and carotid arteries. These are termed "clinically silent" because the person having the infarction does not notice the problem and does not seek medical help, or when they do, physicians do not recognize what has happened.

"Hypertension" (HTN) or high blood pressure, sometimes called arterial hypertension, is a chronic medical condition in which the blood pressure in the arteries is elevated. This requires the heart to work harder than normal to circulate blood through the blood vessels. Blood pressure is summarised by two measurements, systolic and diastolic, which depend on whether the heart muscle is contracting (systole) or relaxed between beats (diastole). Normal blood pressure at rest is within the range of 100-140mmHg systolic (top reading) and 60-90mmHg diastolic (bottom reading). High blood pressure is said to be present if it is persistently at or above 140/90 mmHg (measured at a resting state). Hypertension is classified as either primary (essential) hypertension or secondary hypertension; about 90-95% of cases are categorized as "primary hypertension" which means high blood pressure with no obvious underlying medical cause. The remaining 5-10% of cases (secondary hypertension) are caused by other conditions (e.g. arthero-sclerosis) that affect the kidneys, arteries, heart or endocrine system. Hypertension is a major risk factor for stroke, myocardial infarction (heart attacks), heart failure, aneurysms of the arteries (e.g. aortic aneurysm), peripheral arterial disease and is a cause of chronic kidney disease. Even moderate elevation of arterial blood pressure is associated with a shortened life expectancy. Dietary and lifestyle changes can improve blood pressure control and decrease the risk of associated health complications, although drug treatment is often necessary in people for whom lifestyle changes prove ineffective or insufficient.

Low-density lipoprotein ("LDL") is one of the five major groups of lipoproteins (chylomicrons, VLDL, IDL, LDL, and HDL) that enable transport of multiple different fat molecules, including cholesterol, within the water-based bloodstream. Studies have shown that higher levels of type-B LDL particles (as opposed to type-A LDL particles) promote health problems and cardiovascular disease. Fasting levels of 160 mg/dl (or about 4 mM) LDL cholesterol or above is associated with increased risk of cardiovascular disease events.

"Diabetes", "Diabetes mellitus", or simply "diabetes", is a group of metabolic diseases in which a person has high blood sugar, either because the pancreas does not produce enough insulin (type I), or because cells do not respond (adequately) to the insulin that is produced (type II, gestational diabetes, etc.). Diabetes patients with hypertension and elevated levels of blood LDL/cholesterol may in particular benefit from IL-21/IL-21Rα antagonist treatment according to the present invention as cardio vascular disease progression can be relatively fast in this type of patients.

The term "treatment", as used herein, refers to the medical therapy of any human or other animal subject in need thereof. Treatment of e.g. cardiovascular diseases may be prophylactic, palliative, symptomatic and/or curative.

As used herein, an "isolated" compound is a compound that has been removed from its natural environment. "A purified" compound is a compound that has been increased in purity, such that it exists in a form that is more pure than it exists in its natural environment.

The term "antibody", "recombinant antibody", "monoclonal antibody" and "mAb" as used herein, is intended to refer to immunoglobulin molecules and fragments thereof according to the invention that have the ability to specifically bind to an antigen. Full-length antibodies comprise four (or more) polypeptide chains, i.e. at least two heavy (H) chains and at least two light (L) chains interconnected by disulfide bonds. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. Variable regions and CDRs in an antibody sequence may be identified by aligning the sequences against a database of known variable regions (frameworks and CDRs are defined according to the Kabat numbering scheme herein - (Kabat, EA, Wu, TT, Perry, HM, et al. Sequences of Proteins of Immunological Interest, Fifth Edition. US Department of Health and Human Services, Public Health Service, National Institutes of Health, NIH Publication No. 91-3242, 1991).

The fragment crystallizable region ("Fc region"/"Fc domain") of an antibody comprises the tail regions of an antibody that interact with cell surface receptors called Fc receptors and some proteins of the complement system. This property allows antibodies to activate the immune system. The Fc domain can, however, comprise amino acid mutations that result in modification of these effector functions. Preferably, a modified Fc domain comprises one or more, preferably all of the following mutations that will result in decreased affinity to certain Fc receptors (L234A, L235E, and G237A) and in reduced C1q-mediated complement fixation (A330S and P331 S), respectively (residue numbering according to the EU index). Such Fc domains will still retain a long *in vivo* circulatory half life.

The other part of an antibody, called the "Fab region"/"Fab domain"/"Fab fragment", contains variable regions that define the specific target that the antibody can bind. Fab fragments can be produced from intact antibodies using well known methods, for example by proteolytic cleavage with enzymes such as papain (to produce Fab fragments) or pepsin (to produce F(ab')₂ fragments). Alternatively, antibody fragments may be produced recombinantly, using standard recombinant DNA and protein expression technologies.

Examples of binding fragments encompassed within the term "antibody" thus include but are not limited to: (i) a Fab fragment, a monovalent fragment consisting of the VL, VH, CL and CH1 domains; (ii) F(ab)2 and F(ab')2 fragments, a bivalent fragment comprising two Fab fragments linked by a disulfide bridge at the hinge region; (iii) a Fd fragment consisting of the VH and CH1 domains; (iv) a scFv fragment consisting of the VL and VH domains of a single arm of an antibody, (v) a dAb fragment (Ward et al., (1989) Nature 341:544-546 ), which consists of a VH domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242:423-426: and Huston et al. (1988) Proc. Natl. Acad. Sci. USA 85:5879-5883). Such single chain antibodies are also intended to be encompassed within the term "antibody". Other forms of single chain antibodies, such as diabodies are also encompassed within the term "antibody". Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (see e.g., Hol-liger, P., et al. (1993) Proc. Natl. Acad. Sci. USA 90:6444-6448; Poljak, R. J., et al. (1994) Structure 2:1121-1123).

It is understood that the antigen may have one or more epitopes comprising (1) inants which consist of single peptide chains, (2) conformational epitopes comprising one or more spatially contiguous peptide chains whose respective amino acid sequences are located disjointedly along polypeptide sequence; and (3) post-translational epitopes which comprise, either in whole or part, molecular structures covalently attached to the antigen after translation, such as carbohydrate groups, or the like.

The terms "human antibody", "human antibodies", as used herein, means antibodies having variable and constant regions derived from human germline immunoglobulin sequences. The human antibodies of the invention may include amino acid residues not encoded by human germline immunoglobulin sequences (e.g., mutations introduced by random or site-specific mutagenesis in vitro or by somatic mutation in vivo), for example in the CDRs and in particular CDR3.

Antibodies in which CDR sequences derived from antibodies originating from another mammalian species (such as e.g. a mouse), have been grafted onto human framework sequences and optionally potentially further engineered by mutagenesis are referred to as "humanized antibodies".

The term "chimeric antibody" or "chimeric antibodies" refers to antibodies according to the invention where the light and heavy chain genes have been constructed, typically by genetic engineering, from immunoglobulin variable and constant region genes belonging to different species. For example, the variable segments of genes from a mouse monoclonal antibody may be joined to human constant segments.

The term "epitope", as used herein, is defined in the context of a molecular interaction between an "antigen binding polypeptide", such as an antibody (Ab), and its corresponding antigen (Ag). Generally, "epitope" refers to the area or region on an Ag to which an Ab specifically binds, i.e. the area or region in physical contact with the Ab. Physical contact may be defined through various criteria (e.g. a distance cut-off of 2-6A, such as 3A, such as 4 A, such as 5A; or solvent accessibility) for atoms in the Ab and Ag molecules. A protein epitope may comprise amino acid residues in the Ag that are directly involved in binding to a Ab (also called the immuno-dominant component of the epitope) and other amino acid residues, which are not directly involved in binding, such as amino acid residues of the Ag which are effectively blocked by the Ab, i.e. amino acid residues within the "solvent-excluded surface" and/or the "footprint" of the Ab.

The epitope for a given antibody (Ab)/antigen (Ag) pair can be described and characterized at different levels of detail using a variety of experimental and computational epitope mapping methods. The experimental methods include mutagenesis, X-ray crystallography, Nuclear Magnetic Resonance (NMR) spectroscopy, Hydrogen deuterium exchange Mass Spectrometry (HX-MS) and various competition binding methods; methods that are known in the art. As each method relies on a unique principle, the description of an epitope is intimately linked to the method by which it has been determined. Thus, depending on the epitope mapping method employed, the epitope for a given Ab/Ag pair may be described differently.

Antibodies that bind to the same antigen can be characterised with respect to their ability to bind to their common antigen simultaneously and may be subjected to "competition binding"/"binning". In the present context, the term "binning" refers to a method of grouping antibodies that bind to the same antigen. "Binning" of antibodies may be based on competition binding of two antibodies to their common antigen in assays based on standard techniques such as surface plasmon resonance (SPR), ELISA or flow cytometry.

An antibody's "bin" is defined using a reference antibody. If a second antibody is unable to bind to an antigen at the same time as the reference antibody, the second antibody is said to belong to the same "bin" as the reference antibody. In this case, the reference and the second antibody competitively bind the same part of an antigen and are coined "competing antibodies". If a second antibody is capable of binding to an antigen at the same time as the reference antibody, the second antibody is said to belong to a separate "bin". In this case, the reference and the second antibody do not competitively bind the same part of an antigen and are coined "non-competing antibodies".

A "neutralizing" antibody according to the invention is an antibody having the ability to significantly inhibit/reduce signaling through the IL-21Rα. Neutralizing effects can be assessed in various functional assays using cells expressing IL-21R and γC, e.g. B cell proliferation assays as disclosed in WO2012098113.

The term "affinity", as used herein, defines the strength of the binding of an antibody to an epitope. The affinity of an antibody is measured by the equilibrium dissociation constant KD, defined as [Ab] x [Ag] / [Ab-Ag] where [Ab-Ag] is the molar concentration of the antibodyantigen complex, [Ab] is the molar concentration of the unbound antibody and [Ag] is the molar concentration of the unbound antigen at equilibrium. KD can also be described from the kinetics of complex formation and dissociation, determined by e.g. the SPR method. The rate constants corresponding to the association and the dissociation of a monovalent complex are referred to as the association rate constant ka (or kₒₙ) and dissociation rate constant kd (or k_{off}), respectively. KD is then related to ka and kd through the equation KD = kd / ka. The affinity constant KA is defined by 1/ KD. Preferred methods for determining antibody specificity and affinity by competitive inhibition can be found in Harlow, et al., Antibodies: A Laboratory Manual, Cold Spring Harbor Laboratory Press, Cold Spring Harbor, N.Y., 1988), Colligan et al., eds., Current Protocols in Immunology, Greene Publishing Assoc. and Wiley Interscience, N.Y., (1992, 1993), and Muller, Meth. Enzymol. 92:589-601 (1983). Use of high affinity antibodies are preferred in connection with the present invention, e.g. antibodies binding specifically to the antigen with a binding affinity as measured by e.g. SPR of e.g. 10⁷ M⁻¹ or greater, 10⁸ M⁻¹ or greater, 10⁹ M⁻¹ or greater, 10¹⁰ M⁻¹ or greater, 10¹¹ M⁻¹ or greater, or 10¹² M⁻¹ or greater.

The antibody or fragment thereof may be modified in order to increase its serum half-life, for example, by conjugating with "half life extending moieties" - such as fatty acids or fatty acid derivates, PEG (poly ethylene glycol) or other water soluble polymers, including polysaccharide polymers and peptide derived polymers to increase the half-life. "Half life extending moieties" may also be in the form of peptides, preferably in the form of fusion proteins. Half life extending moieties conjugated to antagonists according to the invention thus include e.g. albumin fused to antibodies and Fc domains fused to antibodies, or fragments thereof, according to the invention. Fusion protein s are preferably made by recombinant techniques.

"Biological drugs"/"biologics" can be composed of sugars, proteins, or nucleic acids or complex combinations of these substances. Biological drugs are isolated from a variety of natural sources (rather than being chemically synthesized) - human, animal, or microorganism - and may be produced by biotechnology methods and other technologies. Biological drugs according to the present invention are preferably derived from recombinant proteins that may optionally be subject to post-translational modification, such as e.g. conjugation with polymeric compounds and or processing to yield fragments of said recombinant proteins (e.g. processing of an antibody to Fab fragments).

"IL-21" has a four helix bundle structure (helix 1-4/A-D - as defined e.g. in fig. 1 in: J. Immunol., 2011 vol. 186 no. 2, p. 708-721), arranged in an up-up-down-down topology typical for the class I cytokines. IL-21 signals through a heterodimeric receptor complex, consisting of the private chain IL-21 Rα (also referred to as IL-21 R) and the γC/IL-2Rγ/common gamma chain the latter being shared by IL-2, IL-4, IL-7, IL-9, and IL-15. γC and IL-21Rα bind to non-overlapping binding sites on IL-21 - IL-21Rα binds to helix 1+3 and γC binds to helix 2+4 on human IL-21. IL-21Rα binds IL-21 with high affinity and provides the

majority of the binding energy. However, interaction with γC is required for signaling and IL-21 mutants which bind IL-21 Rα but fail to interact properly with γC are potent antagonists of IL-21 signaling. IL-21 exerts pleiotropic effects on both innate and adaptive immune responses. It is mainly produced by activated CD4+ T cells, follicular T cells and Natural killer cells. The amino acid sequence of immature human IL-21 is shown in **SEQ ID NO 1.**

IL-21 contributes to inflammation mainly through actions mediated by leukocytes expressing the IL-21 Rα and the γ-chain. Elevated IL-21 levels have been shown to be correlated with arthero-sclerotic lesions. Neutralizing IL-21 and/or IL-21 antibodies thus appear to be an attractive drug type for treatment of cardiovascular diseases in humans either alone or in combination with e.g. ACE inhibitors or other drugs.

IL-21/IL-21Rα antagonists according to the invention are agents with the ability to disrupt/inhibit/reduce IL-21 mediated signalling/effects. Preferred IL-21R antagonists according to the present invention are neutralizing IL-21 antibodies having the ability to compete with either γC or the IL-21 Rα for binding to IL-21.

An example of an IL-21 antibody competing with IL-21 Rα for binding to IL-21 is the "mAb 5" antibody which is a human antibody first disclosed in WO2010055366 as clone number 362.78.1.44. The amino acid sequences of mAb 5 heavy and light chains are shown in **SEQ ID NOs 2+3** (IgG1 isotype version). The mAb 5 antibody binds to helix 1+3 of human IL-21, or more specifically amino acids I37 to Y52 and N92 to P108, as set forth in SEQ ID NO 1. The binding properties of mAb 5, and variants thereof, and their advantages (high affinity and potency), is described in greater detail in WO2012098113. JBC, VOL. 287, NO. 12, pp. 9454-9460, March 16, 2012 discloses further details about IL-21/IL-21Rα binding.

An example of an IL-21 antibody competing with γC for binding to IL-21 is the "mAb 14" antibody which is a human antibody first disclosed in WO2010055366 as clone number 366.328.10.63. "mAb" 14 binds to helix 2+4 of human IL-21. More specifically, mAb 14 binds to Glu 65, Asp 66, Val 67, Glu 68, Thr 69, Asn 70, Glu 72, Trp 73, Lys 117, His 118, Arg 119, Leu 143, Lys 146, Met 147, His 149, Gln 150, and His 151 of human IL-21. The amino acid sequences of mAb 14 heavy and light chains are shown in **SEQ ID NOs 4+5.**

mAb 5 and mAb 14 type of antibodies are generally characterized by having an unusually high affinity (in the nanomolar range) to IL-21 and high potency to neutralize IL-21 induced effects.

Preferred neutralizing IL-21Rα antagonists according to the present invention are those that compete with IL-21 for binding to IL-21Rα. IL-21Rα antagonists according to the invention are preferably antibodies preferably bind the loops connecting the β-strands. IL-21 R antibodies according to the invention bind to the EF loop. Preferred IL-21Rα antibodies according to the invention bind Tyr 29, Gln 52, Gln 54, Tyr 55, Glu 57, Leu 58, Phe 86, His 87, Phe 88, Met 89, Ala 90, Asp 91, Asp 92, Ile 93, Leu 113, Ala 115, Pro 145, Ala 146, Tyr 148, Met 149, Lys 153, Ser 209, Tyr 210 of IL-21R (SEQ ID NO 6).

Preferred IL-21/IL-21Rα antagonists according to the invention are IL-21Rα antibodies that intefrere with binding of IL-21Rα with γC and thus assembly of the IL-21/IL-21Rα/γC complex.

An "ACE inhibitor" (or angiotensin-converting-enzyme inhibitor) is a pharmaceutical drug used primarily for the treatment of hypertension (high blood pressure) and congestive heart failure. Frequently prescribed ACE inhibitors include perindopril, captopril, enalapril, lisinopril, and ramipril. ACE inhibitors are most frequently administered orally.

An IL-21/IL-21Rα antagonist of the invention may be administered parenterally (e.g. intravenously, intramuscularly, subcutaneously or intradermally) and prophylactically and/or therapeutically (on demand).

IL-21/IL-21Rα antagonists according to the invention may be co-administered with one or other more other therapeutic agents. The other agent may be an agent that enhances the effects of the IL-21/ILRα antagonist. The other agent may be intended to treat other symptoms or conditions of the patient. For example, the other agent may be an ACE inhibitor, an analgesic, an immunosuppressant (e.g. methotrexate, dexamethasone, and/or prednisone), an anti-inflammatory agent, antihypertensive drugs, or a statin (HMG-CoA reductase inhibitors). Examples of statins include but are not limited to: atorvastatin (Torvast^{®}, Lipitor^{®}), fluvastatin (Lescol^{®}), lovastatin (Mevacor^{®}, Altocor^{®}, Altoprev^{®}), pitavastatin (Livalo^{®}, Pitava^{®}), pravastatin (Pravachol^{®}, Selektine^{®}, Lipostat^{®}), rosuvastatin (Crestor^{®}) and simvastatin (Zocor^{®}, Lipex^{®}) as well as combination preparations of a statin and another agent.

In case a cardiovascular disease patient is diabetic he or she may preferably also receive treatment with insulin or insulin derivatives/variants and/or one or more other antidiabetic medicines such as e.g. metformin, DPP4 inhibitors, GLP-1, GLP-1 derivatives/variants/analogues, drugs that bind to ATP-dependent K channels on the cell membrane of pancreatic beta cells (sulfonylurea, meglitinides (such as e.g. repaglinide)) etc.

An "ACE inhibitor" (or angiotensin-converting-enzyme inhibitor) is a pharmaceutical drug used primarily for the treatment of hypertension (high blood pressure) and congestive heart failure. Frequently prescribed ACE inhibitors include perindopril, captopril, enalapril, lisinopril, and ramipril. ACE inhibitors are most frequently administered orally. Other "antihypertensive drugs" include but are not limited to: diuretics, adrenergic receptor antagonists (alpha and beta blockers), calcium channel blockers, renin inhibitors, angiotensin II receptor antagonists, aldosterone antagonists, vasodilators, and alpha-2 agonists.

Combined administration of two or more agents may be achieved in a number of different ways. The IL-21/IL-21Rα antagonist and the other agent may be administered together in a single composition or in separate compositions as part of a combined therapy.

IL-21/IL-21Rα antagonists according to the invention may be produced by means of recombinant nucleic acid techniques. In general, a cloned wild-type Protein X nucleic acid sequence is modified to encode the desired protein. This modified sequence is then inserted into an expression vector, which is in turn transformed or transfected into host cells.

In another aspect, the present invention provides compositions and formulations comprising IL-21/IL-21Rα antagonists. For example, the invention provides a pharmaceutical composition that comprises one or more antagonists of the invention, formulated together with one or more pharmaceutically acceptable carrier. The use of preservatives, isotonic agents, chelating agents, stabilizers and surfactants in pharmaceutical compositions is well-known to the skilled person. In one embodiment, the pharmaceutical formulation is an aqueous formulation. The term "aqueous formulation" is defined as a formulation comprising at least 50% w/w water. In another embodiment, the pharmaceutical formulation is a freeze-dried formulation, to which the physician or the patient adds solvents and/or diluents prior to use. In a further aspect, the pharmaceutical formulation comprises an aqueous solution of such an antibody, and a buffer, wherein the antibody is present in a concentration from 1 mg/ml or above (such as e.g. 1-200, 1-100, 50-200, 50-150, 50-100, 1, 5, 10, 15, 20, 25, 30, 35, 40, 45, 50, 60, 70, 80, 90, 100, 125, 150, 175, or 200 mg/ml) and wherein said formulation has a pH from about 6.0 to about 8.0, such as e.g. about 6.0, 6.1, 6.2, 6.3, 6.3, 6.4, 6.5, 6.5, 6.6, 6.7, 6.8, 6.7, 6.8, 6.9, 7.0, 7.1, 7.2, 7.3, 7.4, 7.5, 7.6, 7.7, 7.8, 7.8, 7,9, or 8.0.

Compositions comprising compounds according to the invention are preferably parenteral formulations intended for either extravascular (such as e.g. subcutanous (s.c.) or intradermal) or intravenous (i.v.) administration.

### SEQUENCES:

**SEQ ID No 1:** hIL-21 (incl. signal peptide spanning amino acids 1-29 - mAb 5 epitope shown in bold; IL-21Rα binding site shown in underline; amino acid residues forming the mAb 14 epitope shown with lower case letters in italics) 
MRSSPGNMERIVICLMVIFLGTLVHKSSSQGQDRHMIRMRQLIDIVDQLKNYVNDL
VPEFLPAPedvetnCewSAFSCFQKAQLKSANTGNNERIINVSIKKLKRKPPSTNAGRRQkhrLT
CPSCDSYEKKPPKEFLERFKSlLQkmIhqhLSSRTHGSEDS
**SEQ ID No 2:** "mAb 5": light chain (signal peptide omitted - CDR sequences shown in bold/underline - constant region shown in lowercase letters) 
EIVLTQSPGTLSLSPGERATLSCRASQSVSSSYLAWYQQKPGQAPRLLIYGASSR
ATGIPDRFSGSGSGTDFTLTISRLEPEDFAVYYCQQYGSWTFGQGTKVEIKRtvaapsvfifppsd
eqlksgtasvvcllnnfypreakvqwkvdnalqsgnsqesvteqdskdstyslsstltlskadyekhkvyacevthqglsspvtksf
nrgec
**SEQ ID No** 3: "mAb 5": heavy chain of the IgG1 isotype (signal peptide omitted - CDR sequences shown in bold/underline - constant region shown in lowercase letters) 
QVQLVESGGGVVQPGRSLRLSCAASGFTFSSYGMHWVRQAPGKGLEWVAFIWY
DGSDKYYADSVKGRFTISRDNSKNTLYLQMNSLRAEDTAVYYCARDGDSSDWYGDYYFG
MDVWGQGTTVTVSSastkgpsvfplapsskstsggtaalgclvkdyfpepvtvswnsgaltsgvhtfpavlqssglyslss
vvtvpssslgtqtyicnvnhkpsntkvdkkvepkscdkthtcppcpapeaegapsvflfppkpkdtlmisrtpevtcvvvdvshe
dpevkfnwyvdgvevhnaktkpreeqynstyrvvsvltvlhqdwlngkeykckvsnkalpssiektiskakgqprepqvytlpps
rdeltknqvsltclvkgfypsdiavewesngqpennykttppvldsdgsfflyskltvdksrwqqgnvfscsvmhealhnhytqksl
slspgk
**SEQ ID No 4:** "mAb14" light chain (signal peptide omitted - CDR sequences shown in bold/underline, constant region shown in lowercase letters) 
AIQLTQSPSSLSASVGDRVTITCRASQDIDSALAWYQQKPGKAPKILIHDASSLESG
VPSRFSGSGSGTDFTLTISSLQPEDFATYYCQQFNSYPYTFGQGTKLEIKRtvaapsvfifppsdeq
 
lksgtasvvcllnnfypreakvqwkvdnalqsgnsqesvteqdskdstyslsstltlskadyekhkvyacevthqglsspvtksfnr
gec
SEQ **ID** No 5: "mAb14" heavy chain of the IgG4 isotype (signal peptide omitted - CDR sequences shown in bold/underline, constant region shown in lowercase letters) 
EVQLVESGGGLVKPGGSLRLSCAASGFIFSSYSMNWVRQAPGKGLEWVSSITSGS
YYIHYADSVKGRFTISRDNAKNSLYLQMNSLRAEDTAVYYCVRERGWGYYGMDVWGQGT
TVTVSSastkgpsvfplapcsrstsestaalgclvkdyfpepvtvswnsgaltsgvhtfpavlqssglyslssvvtvpssslgtkty
tcnvdhkpsntkvdkrveskygppcpscpapeflggpsvflfppkpkdtlmisrtpevtcvvvdvsqedpevqfnwyvdgvevh
naktkpreeqfnstyrvvsvltvlhqdwlngkeykckvsnkglpssiektiskakgqprepqvytlppsqeemtknqvsltclvkgf
ypsdiavewesngqpennykttppvldsdgsfflysrltvdksrwqegnvfscsvmhealhnhytqkslslslgk
SEQ **ID** No 6: IL-21Rα (incl. signal sequence) 
MPRGWAAPLLLLLLQGGWGCPDLVCYTDYLQTVICILEMWNLHPSTLTLTWQDQY
EELKDEATSCSLHRSAHNATHATYTCHMDVFHFMADDIFSVNITDQSGNYSQECGSFLLAE
SIKPAPPFNVTVTFSGQYNISWRSDYEDPAFYMLKGKLQYELQYRNRGDPWAVSPRRKLIS
VDSRSVSLLPLEFRKDSSYELQVRAGPMPGSSYQGTWSEWSDPVIFQTQSEELKEGWNPH
LLLLLLLVIVFIPAFWSLKTHPLWRLWKKIWAVPSPERFFMPLYKGCSGDFKKWVGAPFTGS
SLELGPWSPEVPSTLEVYSCHPPRSPAKRLQLTELQEPAELVESDGVPKPSFWPTAQNSG
GSAYSEERDRPYGLVSIDTVTVLDAEGPCTWPCSCEDDGYPALDLDAGLEPSPGLEDPLLD
AGTTVLSCGCVSAGSPGLGGPLGSLLDRLKPPLADGEDWAGGLPWGGRSPGGVSESEAG
SPLAGLDMDTFDSGFVGSDCSSPVECDFTSPGDEGPPRSYLRQWVVIPPPLSSPGPQAS
SEQ **ID** No 7: γC (Common gamma chain/IL-2Rγ) incl. signal sequence

### LIST OF EMBODIMENTS:

1. An IL-21/IL-21Rα antagonist for use in treating cardiovascular diseases.
2. An IL-21/IL-21Rα antagonist for use in treating arthero-sclerosis.
3. An IL-21/IL-21Rα antagonist for use in treating high blood pressure (hypertension).
4. An IL-21/IL-21Rα antagonist for use in treating cardiovascular disease/arthero-sclerosis in a diabetic patient, wherein said patient has hypertension (140/90 mmHg or above, 150/90 mmHg or above, 160/90 mmHg or above, 170/90 mmHg or above, 140/100 mmHg or above, 140/110 mmHg or above, 150/100 mmHg or above, 160/100 mmHg or above, 160/110 mmHg or above) and/or elevated (fasting) blood levels of LDL/cholesterol (3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0 mM or above).
5. An IL-21/IL-21Rα antagonist according to the invention, wherein said antagonist is a biological drug.
6. An IL-21/IL-21Rα antagonist according to the invention, wherein said biological drug is a neutralizing IL-21/IL-21Ra antibody.
7. An IL-21 antibody according to the invention, wherein said antibody is an IL-21 antibody that competes with IL-21Rα for binding to IL-21.
8. An IL-21 antibody according to the invention, wherein said antibody binds to helix 1+3 of human IL-21.
9. An IL-21 antibody according to the invention, wherein said antibody binds to a discontinuous epitope on IL-21, wherein said epitope comprises amino acids I37 to Y52 and N92 to P108 as set forth in SEQ ID NO 1.
10. An IL-21 antibody according to the invention, wherein said antibody comprises the three CDR sequences as set forth in SEQ ID NO 2 and the three CDR sequences as set forth in SEQ ID NO 3.
11. An IL-21 antibody according to the invention, wherein said antibody comprises at least one (e.g. 2, 3, 4, 5, 6, 7, 8, 9, or 10) substitution/deletions/insertions in at least one (e.g. 2, 3, 4, 5, or 6) of the 6 CDR sequences as set forth in SEQ ID NO 2 and SEO ID NO 3.
12. An IL-21 antibody according to the invention, wherein said antibody comprises the VL and the VH sequences as set forth in SEQ ID NO 2 and SEQ ID NO 3.
13. An IL-21 antibody according to the invention, wherein said antibody comprises the heavy and light chain sequences as set forth in SEQ ID NO 2 and SEQ ID NO 3.
14. An IL-21 antibody according to the invention, wherein said antibody is an IL-21 antibody that competes with γC for binding to IL-21.
15. An IL-21 antibody according to the invention, wherein said antibody binds to helix 2+4 of human IL-21.
16. An IL-21 antibody according to the invention, wherein said antibody binds to an epitope comprising amino acids Glu 65, Asp 66, Val 67, Glu 68, Thr 69, Asn 70, Glu 72, Trp 73, Lys 117, His 118, Arg 119, Leu 143, Lys 146, Met 147, His 149, Gln 150, and His 151 as set forth in SEQ ID NO.1
17. An IL-21 antibody according to the invention, wherein said antibody comprises the three CDR sequences as set forth in SEQ ID NO 4 and the three CDR sequences as set forth in SEQ ID NO 5.
18. An IL-21 antibody according to the invention, wherein said antibody comprises at least one (such as e.g. 2, 3, 4, 5, 6, 7, 8, 9, or 10) substitutions/deletions/insertions in at least one ( e.g. 2, 3, 4, 5, or 6) of the 6 CDR sequences as set forth in SEQ ID NO 4 and SEO ID NO 5.
19. An IL-21 antibody according to the invention, wherein said antibody comprises the VL and the VH sequence as set forth in SEQ ID NO 4 and SEQ ID NO 5.
20. An IL-21 antibody according to the invention, wherein said antibody comprises the heavy and light chains as set forth in SEQ ID NO 4 and SEQ ID NO 5.
21. An antibody according to the invention, wherein said antibody is a neutralizing IL-21 Rα antibody that competes with IL-21 for binding to IL-21Rα.
22. An antibody according to the invention, wherein said antibody binds to an epitope comprising amino acids Tyr 29, Gln 52, Gln 54, Tyr 55, Glu 57, Leu 58, Phe 86, His 87, Phe 88, Met 89, Ala 90, Asp 91, Asp 92, Ile 93, Leu 113, Ala 115, Pro 145, Ala 146, Tyr 148, Met 149, Lys 153, Ser 209, Tyr 210 as set forth in SEQ ID NO 6.
23. An antibody according to the invention, wherein said antibody specifically binds to IL-21 with a binding affinity of 10⁷ M⁻¹ or greater.
24. An antibody according to the invention, wherein said antibody specifically binds to IL-21 with a binding affinity of 10⁸ M⁻¹ or greater.
25. An antibody according to the invention, wherein said antibody specifically binds to IL-21 with a binding affinity of 10⁹ M⁻¹ or greater.
26. An antibody according to the invention, wherein said antibody specifically binds to IL-21 with a binding affinity of 10¹⁰ M⁻¹ or greater.
27. An antibody according to the invention, wherein said antibody specifically binds to IL-21 with a binding affinity of 10¹¹ M⁻¹ or greater.
28. An antibody according to the invention, wherein said antibody specifically binds to IL-21 with a binding affinity of 10¹² M⁻¹ or greater.
29. An antibody according to the invention, wherein said antibody is co-administered with one or more ACE inhibitors.
30. An antibody according to the invention, wherein said antibody is co-administered with insulin or an insulin derivative.
31. An antibody according to the invention, wherein said antibody is co-administered with metformin.
32. An antibody according to the invention, wherein said antibody is co-administered with one or more dipeptidyl peptidase-4 inhibitors (DPP-4 inhibitor) such as e.g. sitagliptin.
33. An antibody according to the invention, wherein said antibody is co-administered with GLP-1 or a GLP-1 derivative/analogue, such as e.g. liraglutide.
34. An antibody according to the invention, wherein said antibody is co-administered with a meglitinide such as e.g. repaglinide.
35. An antibody according to the invention, wherein said antibody is co-administered with one or more statins.
36. A pharmaceutical composition comprising an IL-21/IL-21Rα antagonist according to the invention (preferably an antibody) for use in treating cardiovascular diseases.
37. A pharmaceutical composition according to the invention, wherein said composition is an aqueous composition.
38. A pharmaceutical composition according to the invention, wherein said composition comprises at least one pharmaceutically acceptable carrier.
39. An aqueous pharmaceutical composition according to the invention, wherein said composition is for extravascular administration (e.g. subcutaneous or intradermal administration) or for i.v. administration.
40. An IL-21/IL-21Rα antagonist (preferably an antibody) according to the invention for manufacture of a medicament for treatment of cardiovascular diseases and arthero-sclerosis.
41. A method of treating cardiovascular diseases, wherein said method comprises administration to a patient in need thereof an appropriate amount of an IL-21/IL-21Rα antagonist.
42. A method of treating arthero-sclerosis in a diabetic patient, wherein said diabetic patient has hypertension (140/90 mmHg or above) and/or elevated blood levels of LDL/cholesterol (4 mM or above), wherein said method comprises administration to a patient in need thereof an appropriate amount of an IL-21 antibody, wherein said IL-21 antibody competes with a receptor for binding to IL-21, wherein said receptor is selected from the list consisting of: IL-21Rα and γC
43. A method of treatment according to the invention, wherein said IL-21/IL-21Rα antagonist is a neutralizing IL-21 antibody that competes with IL-21Rα for binding to IL-21.
44. A method of treatment according to the invention, wherein said IL-21 antibody binds to a discontinuous epitope on IL-21, wherein said epitope comprises amino acids I37 to Y52 and N92 to P108 as set forth in SEQ ID NO 1.
45. A method of treatment according to the invention, wherein said antagonist is a neutralizing IL-21 antibody that competes with γC for binding to IL-21.

### EXAMPLES

### Generation of Bone marrow derived macrophages from mouse

BMDM are generated from C57/B6 mice (Taconic, DK). The mice are sacrificed by cervical dislocation. Femur and tibia are rinsed and cut open in both ends. The bone marrow is flushed out in PBS with a 25G syringe. Cells are passed through a filter to obtain single cells and plated at 8 x 105 cells/ml in non-coated 10 cm dished in 70% RPMI (Gibco 1640 31870) containing 20% FCS, 1% Pyruvate, 1% Glutamine, 1% Pen/Strep and 1% non essential amino acids and 30% conditioned media (DMEM, 10% FCS) from L929 fibroblasts (Sigma). All cell culture reagents are obtained from Invitrogen. Media was changed every 3-4 days. The cells are differentiated into macrophages after 7 days, and used between day 7 and day 10. For hypoxic incubations cell are seeded in uncoated 6 well plates (Sarsted, GE) and incubated in an InVivo400 hypoxic workstation (Ruskinn, UK) at 0.5% oxygen tension for 24 hrs.

### Foam cell generation

For LPS treatment (Sigma L5543), cells are incubated under serum free conditions 24 hrs. For foam cell formation cells are incubated for 24 hrs with oxLDL (lintracel, USA) in standard RPMI without FCS but with 1.5 % fatty acid free BSA (Sigma). For IL-21 treatment cells are incubated with 200 ng/ml IL-21 (R&D) for 30 min and 2 hrs.

### Endothelial culture

HASMC (LifeTechnologies) are grown in Medium 231 (Cat.no: M-231-500) supplemented with SMGS ( Cat no: S-007-25). At passage 6 cells are seed in 6-wells plates. After 24 hours cell are starved for 24 hrs in medium 231 with 0.1 % serum before 6 hours incubation with either LPS (100ng/ml), oxLDL (25 ug/ml) or control media in duplicates.

### In vivo mRNA expression analysis

Apoprotein E knock out (ApoE KO) (Taconic, DK) are kept on chow diet for 12 week (control (n=1)) and put on high fat diet for another 12 weeks (plaque (n=2)). After sacrifice, the aortas are isolated, rinsed of associated fat and snap frozen in liquid nitrogen.

### mRNA expression analysis

RNA from cell was extracted RNAeasy Kit (Qiagen). For aorta RNA, aortas are homogenized in TRIzol Reagent® (LifeTechnologies) in a TissueLyser (Qiagen) at 2min at 20 1/s. Here after RNA was extracted on RNAeasy column (Qiagen). RNA quality was confirmed by BioAnalyzer analysis (Agilent) before cDNA generation (TaqMan Kit N808-0234 (Perkin Elmer)).

QPCR was performed on 8ng (4 ng for aortic RNA) with murine or human probe sets (Applied Biosystems, DK): PPIA: Mm0234230_g1, SOCS3: Hs00545913_s1, IL-21 : Mm00517640_m1, IL-21R: Mm00600319_m1, IL2RG: Mm00442885_m1, IL17Rc: m00661861_m1, IL17Ra: Mm00434214_m1 and VEGFa: Mm01281449_m1 for murine samples and PPIA: Hs04194521_s1, IL-21R: Hs00222310_m1, IL2RG: Hs00953624_m1 for human samples. Standard curves generated from pooled cDNA was used for quantification, except for murine aortas are quantification was done by the ΔΔCt-method.

### Flow cytometric analysis

For FACS analysis, cell are washed in PBS and blocked with CD16/Cd32 (BD Pharmingen, cat 553142) for 15 min at 4°C before incubation with IL-21R antibody (FAB 5961p R&D) for 30 min at 4°C. Cells are analysed on a Fortessa FACS machine under standard settings and analyzed with.FACSDiva software.

### ApoE aorta FACS analysis

Apo-E -/- mice are purchased from Taconic at 6 weeks of age and housed according to standard rules at the Novo Nordisk A/S animal facility (Måløv, Denmark). At 8 weeks of age, littermate or age-matched male and female apo-E-/- mice (n = 36/group) either received a Western diet (0.21% cholesterol without sodium cholate, D12079B, Research Disets NJ) or a standard rodent chow diet (NIH #31, Taconic). At 12 and 16 weeks of age, mice are euthanized and blood was collected by cardiac puncture with PBS containing 2% heparin to remove blood from all vessels.

### Isolation of murine aortas

The aorta and adventitia was carefully dissected free from adipose tissue and para-aortic lymph nodes. The whole aorta including aortic arch, ascending, descending, thoracic, and abdominal portions are collected and placed in a collection tube with PBS.

### Preparation of single-cell suspensions from aortas and fat tissue

Harvested aortas are micro-dissected and digested with 125 U/ml collagenase type XI, 60 U/ml hyaluronidase type I-s, 60 U/ml DNase1, and 0,5 mg/ml collagenase type I in PBS at 37°C for 1 h. Finally single cell suspension was obtained by mashing the aorta through a 70-µm cell strainer. Single cells from fat tissue are generated by incubating the fat tissue in Liberase TM at 37°C for 30 minutes followed by homogenization by repeated gentle pipetting and mashed through a 70 um cell strainer.

### Flow cytometry of mouse aorta

The resulting aortic single cell suspension was subjected to flow cytometric analysis using the following antibodies: F4/80 efluor450 (eBioscience, clone BM8), CD68 PE (AbD Serotec clone FA-11), Galectin 3 A647 (Biolegend, clone M3/38), CD206 A488 (AbD Serotec, clone MR5D3), CD11c PE Cy7 (BD Pharmingen, clone HL3), CD45 APCefluor780 (BD Pharmingen, clone RM4-5). The adipose tissue cell suspension was stained with following antibodies: IL-21Rα PE (R&D Biosystems, clone 155516), CD4 A647 (BD Pharmingen, clone RM4-5), CCR6 PeCy7 (Biolegend, clone 29-2L17), CD45 APCefluor780 (eBioscience, clone 30-F11). Samples are analyzed on a BD Fortessa using FACS Diva software (BD Biosciences).

### Isolation of lymphocytes from the draining lymphnodes to aorta

Apoprotein E knock out (ApoE KO) (Taconic, DK) are kept on chow diet or on high fat diet for 12 week. After sacrifice, the lymph nodes are passed through a 40 µm cell strainer.

### FACS analysis of lymph node cells

Cells are then cultured for 4 hours in RPMI1640 supplemented with 10 % fetal calf serum, 100 units of penicillin and 100 ug of streptomycin (both Gibco), 20 ng/ml phorbol 12-myristate 13-acetate (Sigma) and 0.5 µg/ml ionomycin (Sigma). Ten µg/ml Brefeldin A (Cell Signalling) was added for the last 2 hours. Cells are harvested and stained with anti-CD4-Pacific blue (Biolegend, clone RM4-5). Cells are then fixed and permeabilized (Cytofix/Cytoperm, BD Biosciences) and stained with anti-IL-21-PE (R&D Systems, clone 149204) and anti-IL-17A-AlexaFluor647 (Biolegend, clone TC11-18H10.1). The percentages of IL-21- and IL17A-expressing cells are analysed on a BD Fortessa (BD Biosciences).

### Results

### Expression of IL-21Rα in vitro

We have found a functional IL-21 receptor pair (IL-21 R and IL2Rg) on murine bone marrow derived macrophages (BMDM) by QPCR. The receptor levels are moderately upregulated by hypoxia (0.5%). IL-21R on BMDM was also analysed by FACS. IL-21R was significantly upregulated by oxLDL treatment for 24 hrs in a dose dependent manner (0, 10, 25 and 50 ug/ml oxLDL). Human aorta smooth muscle cells (HASMC) was stimulated with LPS (100ng/ml) or oxLDL (25 ug/ml) for 6 hrs. IL-21R was expressed at moderate levels at the mRNA level.

### Signaling in BMDM

IL-21 stimulation of BMDM induced a SOCS3 response on RNA level indicating a functional response to IL-21 in BMDM.

### In vivo expression

RNA was extracted form 24 week old murine ApoE KO mice with atherosclerotic plaques (12 weeks of high fat diet) and control mice (ApoE KO 12 weeks old). IL-21Rα and γC are both expressed in aortas with plaque and in non-plaque aorta. Expression of IL-21Rα and γC are markedly upregulated in atherosclerotic aortas compared to control. IL17Ra and IL17Rb are present in both control and atherosclerotic aortas at equal level.

### Murine peri-aortic adipose tissue T cells express IL-21Rα

Both aortic immune cells in Apo-E-/- as well as peri-aortic adipose tissue CD4+ T cells express IL-21Rα. Expression was not markedly regulated by diet. Expression was also noted on additional cells belonging to a macrophage subset. Detailed evaluation showed that the macrophage subset increasing the most in the atherosclerotic plaque is the M2 alternatively activated macrophage expressing CD206.

### Aortic lymphnode T cells express IL-21

The aortic lymphnodes from chow fed ApoE mice and HFD fed ApoE mice had a similar cellularity. The number of CD4 T cells was also similar regardless feeding. Chow feed ApoE mice CD4 T cells expresses IL-21 to a low expression level as a population and ∼1% of the T cells are to be considered as high expressors. In contrast, the basal expression of IL-21 in the HFD ApoE mouse is shifted indicating an elevated capacity to release IL-21. The frequency of high producers also increased to 1.5% of the population.

### Other experiments

Instable atherosclerotic plaques are associated with a necrotic core, a high lipid content and a thin fibrotic plaque. IL-21 can mediate a pro-atherogenic effect either directly or through TH17 cells. IL-21 may have an effect on plaque progression independent of IL17 effects and thus inhibition IL-21 will have anti-atherosclerotic effect on two levels. Data documenting that IL-21 receptors are present on macrophages and foam cells as well as in murine atherosclerotic plaques, supports this hypothesis.

Relative expression levels of IL-21Rα and γC in aortas from ApoE mice will be determined by qPCR to confirm that IL-21Rα is upregulated in atherosclerotic lesions. Samples will include aortas without plaque (12 week, chow), with intermediate plaques size (28 week, chow) and with severe plaque burden (12 week chow+16 week HFD). As IL-21 is closely related to IL17 expression, the expression of 1L17 and receptors IL17Ra and IL17Rb will also be evaluated in the material.

In vitro macrophages and foam cell response to IL-21 will be investigated further to determine if IL-21 affects macrophage biology in a pro-atherosclerotic manner. It will be investigated if IL-21 treatment of macrophages affects surface expression of scavenger receptors responsible for oxLDL uptake (LOX-1, CD36) or cholesterol efflux transporters (ABCA1 and ABCG1) by FACS analysis. In addition the cytokine profile of IL-21 treated macrophages will be evaluated.

As IL-21 can also mediated effects via promotion of pro-atherogenic TH17 cells the effect of TH17 cells and TH17 conditioned media on macrophage and foam cell biology will also be investigated in vitro. Primary focus will be on inflammatory gene expression and lipid uptake and efflux related proteins by FACS analysis and cell death by apoptosis or necrosis.

Sprouting will be monitored using a mouse aortic ring assay. Thoracic aortic rings will be transferred into a bed of polymerized collagen and overlaid with in the presence of conditioned media from Th17 cells or co-embedded with Th17 differentiated cells. The number of microvessels sprouting from each aortic ring will be counted. Endothelial cell and pericyte cell proliferation will be analyzed.

In vivo efficacy study will be evaluated using anti-IL-21 in a 12-20 week study in ApoE KO and LDLR KO mice. Major end-points that will be included are cholesterol, serum markers on inflammation, FACS of aorta, histology on macrophage content, Trichrom/fibronectin/collagen, necrotic core size and course plaque size. Presence of Tregs versus activated T cells will be evaluated.

## Claims

1. An IL-21 antibody for use in treating arthero-sclerosis in a diabetic patient, wherein said diabetic patient has hypertension and elevated blood levels of LDL/cholesterol, wherein said IL-21 antibody competes with a receptor for binding to IL-21, wherein said receptor is selected from the list consisting of: IL-21Rα and γC.

2. An antibody according to claim 1, wherein said antibody competes with IL-21Rα for binding to IL-21.

3. An antibody according to claim 2, wherein said antibody binds to helix 1 and 3 of human IL-21.

4. An antibody according to claim 3, wherein said antibody binds to a discontinuous epitope on IL-21, wherein said epitope comprises amino acids I37 to Y52 and N92 to P108 as set forth in SEQ ID NO 1.

5. An IL-21 antibody according to claim 4, wherein said antibody comprises the three CDR sequences as set forth in SEQ ID NO 2 and the three CDR sequences as set forth in SEO ID NO 3.

6. An antibody according to claim 1, wherein said antibody is an IL-21 antibody that competes with γC for binding to IL-21.

7. An antibody according to claim 6, wherein said antibody binds to helix 2 and 4 of human IL-21.

8. An antibody according to claim 7, wherein said antibody binds to an epitope comprising amino acids Glu 65, Asp 66, Val 67, Glu 68, Thr 69, Asn 70, Glu 72, Trp 73, Lys 117, His 118, Arg 119, Leu 143, Lys 146, Met 147, His 149, Gln 150, and His 151 as set forth in SEQ ID NO.1

9. An antibody according to claim 8, wherein said antibody comprises the three CDR sequences as set forth in SEQ ID NO 4 and the three CDR sequences as set forth in SEO ID NO 5.

10. A pharmaceutical composition, wherein said composition comprises an antibody according to any one of claims 1-9.

11. An antibody according to any of claims 1-9, wherein said antibody is co-administered with one or more statins.

12. A method of treating arthero-sclerosis in a diabetic patient, wherein said diabetic patient has hypertension and elevated blood levels of LDL/cholesterol, wherein said method comprises administering to a patient in need thereof an appropriate amount of an IL-21 antibody, wherein said IL-21 antibody competes with a receptor for binding to IL-21, wherein said receptor is selected from the list consisting of: IL-21Rα and γC.

13. A method of treatment according to claim 12, wherein said antagonist is an IL-21 antibody that competes with IL-21Rα for binding to IL-21.

14. A method of treatment according to claim 13, wherein said IL-21 antibody binds to a discontinuous epitope on IL-21, wherein said epitope comprises amino acids I37 to Y52 and N92 to P108 as set forth in SEQ ID NO 1.

15. A method of treatment according to claim 12, wherein said antagonist is an IL-21 antibody that competes with γC for binding to IL-21.


SEQUENCE LISTING

<110>  Novo Nordisk A/S

<120>  Treatment of Cardiovascular Diseases

<130>  8658

<160>  7

<170>  PatentIn version 3.5

<210>  1
<211>  162
<212>  PRT
<213>  homo sapiens

<400>  1

Met Arg Ser Ser Pro Gly Asn Met Glu Arg Ile Val Ile Cys Leu Met
1               5                   10                  15

Val Ile Phe Leu Gly Thr Leu Val His Lys Ser Ser Ser Gln Gly Gln
            20                  25                  30

Asp Arg His Met Ile Arg Met Arg Gln Leu Ile Asp Ile Val Asp Gln
        35                  40                  45

Leu Lys Asn Tyr Val Asn Asp Leu Val Pro Glu Phe Leu Pro Ala Pro
    50                  55                  60

Glu Asp Val Glu Thr Asn Cys Glu Trp Ser Ala Phe Ser Cys Phe Gln
65                  70                  75                  80

Lys Ala Gln Leu Lys Ser Ala Asn Thr Gly Asn Asn Glu Arg Ile Ile
                85                  90                  95

Asn Val Ser Ile Lys Lys Leu Lys Arg Lys Pro Pro Ser Thr Asn Ala
            100                 105                 110

Gly Arg Arg Gln Lys His Arg Leu Thr Cys Pro Ser Cys Asp Ser Tyr
        115                 120                 125

Glu Lys Lys Pro Pro Lys Glu Phe Leu Glu Arg Phe Lys Ser Leu Leu
    130                 135                 140

Gln Lys Met Ile His Gln His Leu Ser Ser Arg Thr His Gly Ser Glu
145                 150                 155                 160

Asp Ser

<210>  2
<211>  213
<212>  PRT
<213>  artificial

<220>
<223>  mAb 5 light chain

<400>  2

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Trp Thr
                85                  90                  95

Phe Gly Gln Gly Thr Lys Val Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys

    210

<210>  3
<211>  456
<212>  PRT
<213>  artificial

<220>
<223>  mAb 5 heavy chain

<400>  3

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Phe Ile Trp Tyr Asp Gly Ser Asp Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Gly Asp Ser Ser Asp Trp Tyr Gly Asp Tyr Tyr Phe Gly
            100                 105                 110

Met Asp Val Trp Gly Gln Gly Thr Thr Val Thr Val Ser Ser Ala Ser
        115                 120                 125

Thr Lys Gly Pro Ser Val Phe Pro Leu Ala Pro Ser Ser Lys Ser Thr
    130                 135                 140

Ser Gly Gly Thr Ala Ala Leu Gly Cys Leu Val Lys Asp Tyr Phe Pro
145                 150                 155                 160

Glu Pro Val Thr Val Ser Trp Asn Ser Gly Ala Leu Thr Ser Gly Val
                165                 170                 175

His Thr Phe Pro Ala Val Leu Gln Ser Ser Gly Leu Tyr Ser Leu Ser
            180                 185                 190

Ser Val Val Thr Val Pro Ser Ser Ser Leu Gly Thr Gln Thr Tyr Ile
        195                 200                 205

Cys Asn Val Asn His Lys Pro Ser Asn Thr Lys Val Asp Lys Lys Val
    210                 215                 220

Glu Pro Lys Ser Cys Asp Lys Thr His Thr Cys Pro Pro Cys Pro Ala
225                 230                 235                 240

Pro Glu Ala Glu Gly Ala Pro Ser Val Phe Leu Phe Pro Pro Lys Pro
                245                 250                 255

Lys Asp Thr Leu Met Ile Ser Arg Thr Pro Glu Val Thr Cys Val Val
            260                 265                 270

Val Asp Val Ser His Glu Asp Pro Glu Val Lys Phe Asn Trp Tyr Val
        275                 280                 285

Asp Gly Val Glu Val His Asn Ala Lys Thr Lys Pro Arg Glu Glu Gln
    290                 295                 300

Tyr Asn Ser Thr Tyr Arg Val Val Ser Val Leu Thr Val Leu His Gln
305                 310                 315                 320

Asp Trp Leu Asn Gly Lys Glu Tyr Lys Cys Lys Val Ser Asn Lys Ala
                325                 330                 335

Leu Pro Ser Ser Ile Glu Lys Thr Ile Ser Lys Ala Lys Gly Gln Pro
            340                 345                 350

Arg Glu Pro Gln Val Tyr Thr Leu Pro Pro Ser Arg Asp Glu Leu Thr
        355                 360                 365

Lys Asn Gln Val Ser Leu Thr Cys Leu Val Lys Gly Phe Tyr Pro Ser
    370                 375                 380

Asp Ile Ala Val Glu Trp Glu Ser Asn Gly Gln Pro Glu Asn Asn Tyr
385                 390                 395                 400

Lys Thr Thr Pro Pro Val Leu Asp Ser Asp Gly Ser Phe Phe Leu Tyr
                405                 410                 415

Ser Lys Leu Thr Val Asp Lys Ser Arg Trp Gln Gln Gly Asn Val Phe
            420                 425                 430

Ser Cys Ser Val Met His Glu Ala Leu His Asn His Tyr Thr Gln Lys
        435                 440                 445

Ser Leu Ser Leu Ser Pro Gly Lys

450                 455

<210>  4
<211>  214
<212>  PRT
<213>  artificial

<220>
<223>  mAb 14 light chain

<400>  4

Ala Ile Gln Leu Thr Gln Ser Pro Ser Ser Leu Ser Ala Ser Val Gly
1               5                   10                  15

Asp Arg Val Thr Ile Thr Cys Arg Ala Ser Gln Asp Ile Asp Ser Ala
            20                  25                  30

Leu Ala Trp Tyr Gln Gln Lys Pro Gly Lys Ala Pro Lys Ile Leu Ile
        35                  40                  45

His Asp Ala Ser Ser Leu Glu Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Phe Asn Ser Tyr Pro Tyr
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  5
<211>  447
<212>  PRT
<213>  artificial

<220>
<223>  mAb 14 heavy chain

<400>  5

Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Ile Phe Ser Ser Tyr
            20                  25                  30

Ser Met Asn Trp Val Arg Gln Ala Pro Gly Lys Gly Leu Glu Trp Val
        35                  40                  45

Ser Ser Ile Thr Ser Gly Ser Tyr Tyr Ile His Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Val Arg Glu Arg Gly Trp Gly Tyr Tyr Gly Met Asp Val Trp Gly Gln
            100                 105                 110

Gly Thr Thr Val Thr Val Ser Ser Ala Ser Thr Lys Gly Pro Ser Val
        115                 120                 125

Phe Pro Leu Ala Pro Cys Ser Arg Ser Thr Ser Glu Ser Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Lys Thr Tyr Thr Cys Asn Val Asp His Lys
        195                 200                 205

Pro Ser Asn Thr Lys Val Asp Lys Arg Val Glu Ser Lys Tyr Gly Pro
    210                 215                 220

Pro Cys Pro Ser Cys Pro Ala Pro Glu Phe Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser Gln Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Gly Leu Pro Ser Ser Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Gln Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Arg Leu Thr Val Asp Lys Ser Arg
                405                 410                 415

Trp Gln Glu Gly Asn Val Phe Ser Cys Ser Val Met His Glu Ala Leu
            420                 425                 430

His Asn His Tyr Thr Gln Lys Ser Leu Ser Leu Ser Leu Gly Lys
        435                 440                 445

<210>  6
<211>  538
<212>  PRT
<213>  homo sapiens

<400>  6

Met Pro Arg Gly Trp Ala Ala Pro Leu Leu Leu Leu Leu Leu Gln Gly
1               5                   10                  15

Gly Trp Gly Cys Pro Asp Leu Val Cys Tyr Thr Asp Tyr Leu Gln Thr
            20                  25                  30

Val Ile Cys Ile Leu Glu Met Trp Asn Leu His Pro Ser Thr Leu Thr
        35                  40                  45

Leu Thr Trp Gln Asp Gln Tyr Glu Glu Leu Lys Asp Glu Ala Thr Ser
    50                  55                  60

Cys Ser Leu His Arg Ser Ala His Asn Ala Thr His Ala Thr Tyr Thr
65                  70                  75                  80

Cys His Met Asp Val Phe His Phe Met Ala Asp Asp Ile Phe Ser Val
                85                  90                  95

Asn Ile Thr Asp Gln Ser Gly Asn Tyr Ser Gln Glu Cys Gly Ser Phe
            100                 105                 110

Leu Leu Ala Glu Ser Ile Lys Pro Ala Pro Pro Phe Asn Val Thr Val
        115                 120                 125

Thr Phe Ser Gly Gln Tyr Asn Ile Ser Trp Arg Ser Asp Tyr Glu Asp
    130                 135                 140

Pro Ala Phe Tyr Met Leu Lys Gly Lys Leu Gln Tyr Glu Leu Gln Tyr
145                 150                 155                 160

Arg Asn Arg Gly Asp Pro Trp Ala Val Ser Pro Arg Arg Lys Leu Ile
                165                 170                 175

Ser Val Asp Ser Arg Ser Val Ser Leu Leu Pro Leu Glu Phe Arg Lys
            180                 185                 190

Asp Ser Ser Tyr Glu Leu Gln Val Arg Ala Gly Pro Met Pro Gly Ser
        195                 200                 205

Ser Tyr Gln Gly Thr Trp Ser Glu Trp Ser Asp Pro Val Ile Phe Gln
    210                 215                 220

Thr Gln Ser Glu Glu Leu Lys Glu Gly Trp Asn Pro His Leu Leu Leu
225                 230                 235                 240

Leu Leu Leu Leu Val Ile Val Phe Ile Pro Ala Phe Trp Ser Leu Lys
                245                 250                 255

Thr His Pro Leu Trp Arg Leu Trp Lys Lys Ile Trp Ala Val Pro Ser
            260                 265                 270

Pro Glu Arg Phe Phe Met Pro Leu Tyr Lys Gly Cys Ser Gly Asp Phe
        275                 280                 285

Lys Lys Trp Val Gly Ala Pro Phe Thr Gly Ser Ser Leu Glu Leu Gly
    290                 295                 300

Pro Trp Ser Pro Glu Val Pro Ser Thr Leu Glu Val Tyr Ser Cys His
305                 310                 315                 320

Pro Pro Arg Ser Pro Ala Lys Arg Leu Gln Leu Thr Glu Leu Gln Glu
                325                 330                 335

Pro Ala Glu Leu Val Glu Ser Asp Gly Val Pro Lys Pro Ser Phe Trp
            340                 345                 350

Pro Thr Ala Gln Asn Ser Gly Gly Ser Ala Tyr Ser Glu Glu Arg Asp
        355                 360                 365

Arg Pro Tyr Gly Leu Val Ser Ile Asp Thr Val Thr Val Leu Asp Ala
    370                 375                 380

Glu Gly Pro Cys Thr Trp Pro Cys Ser Cys Glu Asp Asp Gly Tyr Pro
385                 390                 395                 400

Ala Leu Asp Leu Asp Ala Gly Leu Glu Pro Ser Pro Gly Leu Glu Asp
                405                 410                 415

Pro Leu Leu Asp Ala Gly Thr Thr Val Leu Ser Cys Gly Cys Val Ser
            420                 425                 430

Ala Gly Ser Pro Gly Leu Gly Gly Pro Leu Gly Ser Leu Leu Asp Arg
        435                 440                 445

Leu Lys Pro Pro Leu Ala Asp Gly Glu Asp Trp Ala Gly Gly Leu Pro
    450                 455                 460

Trp Gly Gly Arg Ser Pro Gly Gly Val Ser Glu Ser Glu Ala Gly Ser

465                 470                 475                 480

Pro Leu Ala Gly Leu Asp Met Asp Thr Phe Asp Ser Gly Phe Val Gly
                485                 490                 495

Ser Asp Cys Ser Ser Pro Val Glu Cys Asp Phe Thr Ser Pro Gly Asp
            500                 505                 510

Glu Gly Pro Pro Arg Ser Tyr Leu Arg Gln Trp Val Val Ile Pro Pro
        515                 520                 525

Pro Leu Ser Ser Pro Gly Pro Gln Ala Ser
    530                 535

<210>  7
<211>  369
<212>  PRT
<213>  homo sapiens

<400>  7